# EUROPEAN PATENT SPECIFICATION

(11) **EP 2 671 580 B1**
(45) Date of publication and mention of the grant of the patent: **20.11.2019**
(21) Application number: 11848650.5
(22) Date of filing: 16.12.2011
(51) Int. Cl.: A61K 31/355, A61K 31/375, A61P 25/00

(54) **USE OF ANTIOXIDANTS FOR THE TREATMENT OF COGNITIVE AND BEHAVIOURAL DISORDERS IN INDIVIDUALS WITH FRAGILE X SYNDROME**
VERWENDUNG VON ANTIOXIDANTIEN ZUR BEHANDLUNG VON KOGNITIVEN UND VERHALTENSSTÖRUNGEN BEI PERSONEN MIT FRAGILEM X-SYNDROM
UTILISATION D'ANTIOXYDANTS POUR LE TRAITEMENT DES TROUBLES COGNITIFS ET DU COMPORTEMENT CHEZ DES INDIVIDUS ATTEINTS DU SYNDROME DE L'X FRAGILE

(30) Priority: 17.12.2010 ES 201031870
(43) Date of publication of application: 11.12.2013
(73) Proprietor: Instituto Mediterráneo Para el Avance de la Biotecnologia y la Investigacion Sanitaria (Fundacion IMABIS), 29010 Málaga (ES); Servicio Andaluz de Salud, 41071 Sevilla (ES)
(72) Inventor: DE DIEGO OTERO, María Yolanda, 29010 Málaga (ES); PÉREZ COSTILLAS, Lucia María, 41071 Sevilla (ES); DEL ARCO HERRERA, Ignacio, 29010 Málaga (ES)
(74) Representative: Hoffmann Eitle
(86) International application number: PCT/ES2011/070875
(87) International publication number: WO 2012/080554

(56) References cited:
- EP-A2- 0 836 849
- US-A1- 2002 182 196
- U.S. National Institutes of Health: "Safety and Efficacy Study of Antioxidants for the Treatment of the Fragile X Syndrome (SXF-TRA152)", , 5 April 2011 (2011-04-05), XP002725825, Retrieved from the Internet: URL:http://clinicaltrials.gov/archive/NCT0 1329770/2011_04_05 [retrieved on 2014-06-13]
- LEVENGA J ET AL: "Potential therapeutic interventions for fragile X syndrome", TRENDS IN MOLECULAR MEDICINE, ELSEVIER CURRENT TRENDS, GB, vol. 16, no. 11, 1 November 2010 (2010-11-01), pages 516-527, XP027450768, ISSN: 1471-4914, DOI: 10.1016/J.MOLMED.2010.08.005 [retrieved on 2010-09-21]
- RANDI HAGERMAN ET AL: "Treatment of fragile X-associated tremor ataxia syndrome (FXTAS) and related neurological problems.", CLINICAL INTERVENTIONS IN AGING, vol. 3, no. 2, 1 January 2008 (2008-01-01) , pages 251-262, XP055100210, ISSN: 1176-9092
- EL BEKAY RAJAA ET AL: "Enhanced markers of oxidative stress, altered antioxidants and NADPH-oxidase activation in brains from Fragile X mental retardation 1-deficient mice, a pathological model for Fragile X syndrome.", December 2007 (2007-12), THE EUROPEAN JOURNAL OF NEUROSCIENCE DEC 2007, VOL. 26, NR. 11, PAGE(S) 3169 - 3180, XP002725826, ISSN: 0953-816X * the whole document *
- FINSTERER J. ET AL.: 'Ataxias with autosomal, x-chromosomal or maternal inheritance.' CAN. J. NEUROL. SCI. vol. 36, 2009, pages 409 - 428, XP055118237
- DE DIEGO-OTERO Y. ET AL.: 'Alpha-tocopherol protects against oxidative stress in the fragile X knockout mouse: an experimental therapeutic approach for the Fmrl deficiency.' NEUROPSYCHOPHARMACOLOGY. vol. 34, no. 4, 2009, pages 1011 - 1026, XP055118244
- MAXWELL COLLEEN J ET AL: "Supplemental use of antioxidant vitamins and subsequent risk of cognitive decline and dementia.", 2005, DEMENTIA AND GERIATRIC COGNITIVE DISORDERS 2005, VOL. 20, NR. 1, PAGE(S) 45 - 51 ISSN: 1420-8008
- KAZDOBA TATIANA M ET AL: "Modeling fragile X syndrome in the Fmr1 knockout mouse.", November 2014 (2014-11), INTRACTABLE & RARE DISEASES RESEARCH NOV 2014, VOL. 3, NR. 4, PAGE(S) 118 - 133 ISSN: 2186-3644
- DAVENPORT MATTHEW H ET AL: "Pharmacotherapy for Fragile X Syndrome: Progress to Date.", March 2016 (2016-03), DRUGS MAR 2016, VOL. 76, NR. 4, PAGE(S) 431 - 445 ISSN: 1179-1950

## Description

The present invention belongs to the field of medicine, and relates to the use of antioxidants such as vitamins, polyphenols or the combinations thereof for the treatment of cognitive and behavioural disorders in patients affected with Fragile X syndrome. Specifically, it relates to the use of said vitamins at a given concentration, at therapeutic doses, in order to obtain the greatest possible quantity of antioxidants in all the body tissues to eliminate the excess free radicals produced in the cells thereof.

### STATE OF THE ART

The main cause of hereditary developmental disorders in children that evolve with intellectual disability is Fragile X Syndrome (it affects 1 in 2600 newborn males and 1 in 4000 females) (Fernandez-Carvajal et al. 2009). Despite being one of the most frequent hereditary diseases in childhood, it is still largely unknown amongst the general population and even amongst the professionals involved in its care. This syndrome presents other characteristics, jointly with intellectual disability, such as hypotonia in early childhood, lack of interaction with the mother and the environment, delayed psychomotor development, macroorchidism (large-sized testicles), connective tissue dysplasia (joint hypermobility, mitral valve prolapse), recurrent otitis in childhood, facial anomalies such as elongated face and low-set large, everted ears, strabismus and convulsions; the behavioural characteristics include hyperactivity, anxiety, autistic features, language problems and delay in learning.

In 1969, a fragile site associated with the disease was described, located at the end of the long arm of the X chromosome called FRAXA; this marker gave this pathology a new name; since then, it is called Fragile X Syndrome; prior to this, it was known by the name of doctors Martin and Bel, who had described it in 1943. FMR1, the gene involved in Fragile X syndrome, was cloned in 1991 at the same site where the fragility was located. The CGG trinucleotide repeat expansion is the most frequent mutation in the patients affected. After a number of repeats of 200 CGG is exceeded, hypermethylation of the FMR1 promoter is observed, which inhibits transcription of the gene. The absence of FMRP, the functional protein product of the FMR1 gene, is responsible for the disorder observed in patients. However, the physiological function of the FMRP protein is still under discussion and the mechanisms that might explain the pathogenesis of the syndrome are still unclear. It is known that it acts as an mRNA-binding protein, transporting it from the nucleus to the cytoplasm, and regulates the expression of a part of the messengers that it transports. FMRP forms ribonucleoprotein aggregates, which transport mRNA from the nucleus to the translation area of the protein, in a movement that is dependent on microtubules in PC12 cells stimulated with nerve growth factors (de Diego et al. 2002. Mol Cell Biol. Dec; 22(23): 8332-41).

It has been demonstrated that the expression of the FMRP protein may be subject to a specific regulation of ectodermal tissue. Moreover, a very high expression of FMRP is observed in the suprarenal medulla, without co-expression of FXR1P and FXR2P (two homologous proteins of FMRP). Consequently, it may be deduced that the function of FMRP must be specific in the suprarenal medulla, and that the absence thereof is not compensated by the two other proteins in the family (de Diego et al. 2000. Gene Function & Disease. 2000. 1(1): 28-37).

The alteration of the hypothalamic-pituitary-adrenal (HPA) axis is an underlying explanation for the phenotype of Fragile X Syndrome, but the primary disorder is unkown. The genetic mutation translates into the absence of the FMRP protein in the patients' tissues, including the adrenal medulla, where it has a specific function, modifying hormone secretion in response to stress and altering the integration of the HPA axis involved in the body response to environmental and psychic stress. Previous publications indicate that the lack of FMRP affects the expression of other proteins involved in the cellular REDOX function, such as superoxide dismutase (SOD), glutathione peroxidase (GPx), stearoyl-Coenzyme A desaturase 1U (SCD1), Phosphatidylinositol-3-kinase (Pi3-Kinase p85 subunit) or the GabaA receptor. There are results that endorse the involvement of the excess production of free radicals dependent on NADPH-oxidase and a high oxidative stress in the brain of "Fmr1-knockout" mice, as a part of the physiopathology of the central nervous system in the animal model of Fragile X syndrome (el Bekay et al., 2007. Eur J Neurosci. Dec; 26(11): 3169-80). Subsequently, it has been demonstrated that these biochemical alterations may be reverted in the Fmr1-KO mouse using a chronic treatment with compounds that regulate the oxidative balance, such as tocopherol and melatonin, normalising several parameters characteristic of the syndrome, such as hyperactivity, anxiety disorder and cognitive deficit (de Diego et al., 2009; Romero et al. 2009).

Currently, the pharmacological treatments used in patients have a limited effect on the symptoms that they present. Central nervous system stimulants have been proposed for the treatment of hyperactivity, and antipsychotics have been proposed to treat aggressive and self-injury disorders. Anxiolytic drugs have been proposed to treat anxiety; anticonvulsant drugs are also used in specific cases to treat symptoms such as epilepsy. In general, the treatments used are symptomatic, and there is still no specific approach that prevents the appearance of the symptoms observed in the pathology.

Recent experiments in animal models allow us to think that the treatment with glutamate receptor antagonist drugs, such as the compound MPEP, an antagonist of Glutamate mGlur5 receptors, may lead to the development of a possible experimental treatment of the syndrome. However, the first published attempt at gene therapy trials in the Fmr1-null mouse was not able to normalise the altered parameters, leading to a new mouse model with the pathology. A study published in December 2007 demonstrated that, in Fragile X Syndrome model mice, the symptoms may be reverted, by crossing the Fmr1-knockout mouse with a mutant of the gene of the glutamate mGlur5 receptor. A prior study had likewise demonstrated that the phenotype of the Fmr1-KO mouse may be reverted, generating a second double mutant model, by crossing the Fmr1-KO mouse with a null mutant of the p21 kinase gene.

By the late 1980s, trials had begun to test the effectiveness of Folic Acid or propanolol to treat the Syndrome disorders. However, only in the past 3 years have a number of experimental therapeutic initiatives been published, on the basis of the findings described in various basic research projects that pointed to neurotransmission problems as the physiopathological basis of the syndrome. These studies indicated a potential excess of glutamatergic excitation or a reduction in GABAergic inhibition. Recently, the results of a trial in adults with FXS were published which demonstrate the efficacy of a single dose of Fenoban, a potent, selective glutamatergic antagonist of the mGLUR5 receptor. Also recently, a potential cholinergic dysfunction in Fragile X Syndrome has been proposed, which might be involved in the symptoms and it has been described that the trial with donezepil, an acetylcholinesterase inhibitor, significantly improves cognitive and behavioural functions in affected patients. Another study publishes the results of the trial of the compound Nemantine in 6 affected patients, of which 4 show symptoms of improvement. The safety and efficacy of minocycline in the treatment of behavioural anomalies that appear in human beings with FXS have been evaluated in a reduced number of patients, and significant improvements in behavioural parameters have been observed. The efficacy of melatonin to reduce the sleep problems described in patients with Fragile X syndrome has also been verified. Prior treatment trials have already proven the partial effectiveness of different compounds, such as lithium, which has shown efficacy in improving behaviour and learning, L-acetylcarnitine, which has demonstrated efficacy in reducing hyperactivity and improving social relations in affected children between 6 and 13 years of age, and another study proved the effect of the compound CX516, an AMPA receptor modulator, which demonstrates a certain effectiveness, but presents doubts regarding its ease of use due to a dosage problem.
- Several clinical trials are currently being developed in relation to Fragile X Syndrome or autism (one of the most frequent disorders in the Syndrome). Thus, we may find a trial with vitamins designed to treat the oxidative stress described in autistic children, developed at the research institute of the "Arkansas Children's Hospital". As regards FXS, trials are being developed to test the efficacy of drugs such as STX209 (Arbaclofen) for the treatment of irritability in patients. Or, in another case, Aripiprazole to treat aggressive behaviours in patients. Also, Riluzole for the obsessive-compulsive behaviour in those affected. Another trial under development would be a single dose of the drug NPL-2009, in order to test its safety. Several trials are testing inhibitors of glutamatergic neurotransmission, such as, for example, the compound AFQ056 from Novartis, which since 2008 demonstrates its effectiveness on the behavioural and learning problems of patients. Since 2009, Roche is testing the compound RO4917523 and Merck is testing the effectiveness of the compound STX107.

Currently, despite all the experimental therapeutic approaches, the available pharmacological treatments have a limited effect on the most frequent symptoms in patients affected by the syndrome (Chiurazzi et al. 2003. Curr Opin Pediatr. Dec; 15(6): 559-66). Currently, there is no specific healing treatment available for this pathology, because the available psycho-drugs have a limited effectiveness on the patients' symptoms. Therefore, treatment trials are necessary in order to determine which compounds may be more specific to improve the patients affected, who have severe limitations in cognitive development and behaviour, which will affect their socio-occupational integration in adult life.

### DESCRIPTION OF THE INVENTION

The authors of the present invention have developed an antioxidant composition, and a combined preparation, as defined in the claims to be used in the treatment of the symptoms of Fragile X syndrome in humans.

Therefore, a first aspect of the disclosure relates to a composition which comprises lipophilic antioxidant compounds and hydrophilic antioxidant compounds.

The term "hydrophilic compound" or "hydrophilic molecule" or "polar molecule" refers to any molecule that has affinity for water. These are molecules that may form a transient bond with water (H₂O) by the linkage of hydrogen. This is thermodynamically favourable, and these molecules are not only soluble in water, but in other polar solvents as well. On the contrary, they cannot diffuse across the plasma membrane and interact with cytosol or nucleus receptors; for example, without being limited thereto, peptides such as insulin, or proteins such as the growth hormone, and small charged molecules such as acetylcholine and others derived from some amino acids, such as epinephrine, histamine, serotonin and dopamine, some of which function like hormones or neurotransmitters. In these cases, the effect of the molecule bound to the cell surface is almost immediate, but only persists for a short period of time; however, the effect of some trophic factors may be extended for several days, since it can also regulate the gene expression patterns of the target cell.

The term "lipophilic compound" or "lipophilic molecule" refers to any molecule that is soluble in lipids. They are capable of diffusing across the plasma membrane and interact with cytosol or nucleus receptors; for example, without being limited thereto, steroid hormones, thyroxine and retinoic acid derivatives. After crossing the plasma membrane, these hormones interact with intracellular receptors, forming complexes that are capable of increasing or reducing the transcription of specific genes; these complexes also contribute to the stability of certain messenger RNAs. As a general rule, these compounds exert their effect for hours or days and contribute to the growth and differentiation of specific tissues.

Preferably, the lipophilic molecules have a solubility in water of less than 2.5 µg/ml; more preferably, of less than 2 µg/ml; and, even more preferably, of less than 1 µg/ml.

In a preferred embodiment of this aspect of the disclosure, the quantity of the lipophilic antioxidant compound is at least 150 mg. In another preferred embodiment, the quantity of the hydrophilic antioxidant compound is at least 150 mg. In another preferred embodiment, the quantity of the lipophilic antioxidant compound ranges between 150 and 600 mg. In another preferred embodiment, the quantity of the hydrophilic antioxidant compound ranges between 150 and 800 mg. In another preferred embodiment, the lipophilic antioxidant compound is Tocopherol (Vitamin E). In another preferred embodiment, the hydrophilic antioxidant compound is ascorbic acid (Vitamin C). In another preferred embodiment, the composition further comprises vitamin A. In another more preferred embodiment, the composition further comprises polyphenols and, more preferably, the polyphenols are selected from the list that comprises: resveratrol, quercetin, epigallocatechin gallate, apocynin, or any combination thereof.
In this specification, "polyphenols" is understood to mean any chemical substance characterised by the presence of more than one phenol group per molecule. Preferably, said substances are obtained from plants; more preferably, they are secondary metabolites of the plants of the shikimic acid pathway, and, in general, they are subdivided into hydrolysable tannins, which are gallic acid esters of glucose and other sugars; and phenylpropanoids, such as lignin, flavonoids and condensed tannins. Such as Phenol: kaempferol. Pyrocatechol: catechin, quercetin. Pyrogallol: gallocatechins, tannins, myricetin, epigallocatechin gallate. Resorcinol: Resveratrol. Phloroglucinol: Flavonoids. Hydroquinone: Arbutin.

In another preferred embodiment, the composition further comprises a pharmaceutically acceptable vehicle. In another more preferred embodiment, the composition of the disclosure comprises another active principle.

The lipophilic antioxidant compounds and the hydrophilic antioxidant compounds may be used separately or combined, in order to achieve certain pharmacological effects that currently cannot be achieved with other medicaments.

Therefore, another aspect of the disclosure describes a combined preparation that comprises lipophilic antioxidant compounds and hydrophilic antioxidant compounds.

In a preferred embodiment of this aspect of the disclosure, the quantity of the lipophilic antioxidant compound of the combined preparation is at least 150 mg. In another preferred embodiment, the quantity of the hydrophilic antioxidant compound is at least 150 mg. In another preferred embodiment, the quantity of the lipophilic antioxidant compound ranges between 150 and 600 mg. In another preferred embodiment, the quantity of the hydrophilic antioxidant compound ranges between 150 and 800 mg. In another preferred embodiment, the lipophilic antioxidant compound is Tocopherol (Vitamin E). In another preferred embodiment, the hydrophilic antioxidant compound is ascorbic acid (Vitamin C). In another preferred embodiment, the combined preparation further comprises vitamin A. In another more preferred embodiment, the combined preparation further comprises polyphenols.

In another preferred embodiment, the combined preparation as described herein further comprises a pharmaceutically acceptable vehicle. In another preferred embodiment, the combined preparation further comprises another active principle.

It is worth emphasising that, in this specification, the term "combined preparation", also called "juxtaposition", means that the components of the combined preparation need not be present as a union, for example in a composition, in order to be available for the separate or sequential application thereof. Thus, the expression "juxtaposed" means that it is not necessarily a genuine combination, in view of the physical separation of the components.

Another aspect relates to the use of the composition as described herein for the elaboration of a medicament, or to the separate, simultaneous or sequential use of the active principles of the combined preparation of the disclosure for the elaboration of a medicament. Alternatively, it also relates to the composition of the disclosure to be used as a medicament, or to the combined preparation of the disclosure to be used as a medicament.

Another aspect relates to the use of the composition as described herein for the elaboration of a medicament designed for the treatment of the symptoms of Fragile X syndrome in a mammal, or to the separate, simultaneous or sequential use of the active principles of the combined preparation of the invention for the elaboration of a medicament designed for the treatment of the symptoms of Fragile X syndrome in a mammal. Alternatively, it also relates to the composition of the disclosure for the treatment of the symptoms of Fragile X syndrome in a mammal, or to the combined preparation as described herein for the treatment of the symptoms of Fragile X syndrome in a mammal.

In a preferred embodiment of this aspect of the disclosure, the mammal is a human being. In another preferred embodiment, the human being is under 20 years of age. In another more preferred embodiment, the human being is between 6 and 12 years of age. In another more preferred embodiment, the human being is under 12 years of age. In another more preferred embodiment, the human being is between 13 and 18 years of age.

In another preferred embodiment of this aspect of the disclosure, the symptoms of Fragile X syndrome are: cardiac, neurological, motor, morphological symptoms or any combination thereof. In another preferred embodiment, the symptoms are cognitive and behavioural disorders. In another more preferred embodiment, the cognitive and behavioural disorders are selected from the list that comprises: excessive motor restlessness, need for immediate satisfaction of their demands, arrogance, unpredictable bad temper explosions, limited attention, angry or unsociable appearance, abrupt mood changes, disquiet, impulsiveness, irritability, excessive sensitivity to criticism, or any combination thereof.

Another aspect of the disclosure relates to the use of the lipophilic antioxidant compounds and the hydrophilic antioxidant compounds separately, simultaneously or sequentially, characterised in that a 10-mg/kg/day dose of each compound is administered, with a maximum of 600 mg/day for the lipophilic antioxidant compounds and a maximum of 800 mg/day for the hydrophilic antioxidant compounds. In a preferred embodiment, the lipophilic antioxidant compound is Tocopherol (Vitamin E).

In another preferred embodiment, the hydrophilic antioxidant compound is ascorbic acid (Vitamin C). In another preferred embodiment, the combined preparation of the disclosure further comprises vitamin A. In another more preferred embodiment, the combined preparation of the disclosure further comprises polyphenols and, more preferably, the polyphenols are selected from the list that comprises: epigallocatechin gallate, quercetin, apocynin, resveratrol or any combination thereof. In another preferred embodiment, the administration is performed once a day. In another preferred embodiment, the administration is performed twice a day.

In another preferred embodiment, the combined preparation of the disclosure further comprises a pharmaceutically acceptable vehicle. In another preferred embodiment, the combined preparation of the disclosure further comprises another active principle.

As used herein, the term "active principle", "active substance", "pharmaceutically active substance", "active ingredient" or "pharmaceutically active ingredient" means any component that potentially provides a pharmacological activity or another different effect for the diagnosis, cure, mitigation, treatment, or prevention of a disease, or which affects the structure or function of the body of humans or other animals. The term includes those components that promote a chemical change during the elaboration of the drug and are present therein in an expected modified form that provides the specific activity or effect.

Both the compositions of the present disclosure and the combined preparation may be formulated for administration to an animal, and, more preferably, to a mammal, including a human, in a variety of forms known in the state of the art. Thus, they may be, without being limited thereto, in sterile aqueous solution or in biological fluids, such as serum. The aqueous solutions may or may not be buffered and may or may not have additional active or inactive components. The additional components include salts, to modulate the ionic strength; preservatives, including, without being limited thereto, antimicrobial, antioxidant, chelating agents, and similar; and nutrients, including glucose, dextrose, vitamins and minerals. Alternatively, the compositions may be prepared for administration in solid form. The compositions may be combined with several inert vehicles or excipients, including, without being limited thereto, binders such as microcrystalline cellulose, tragacanth, or gelatin; excipients such as starch or lactose; dispersant agents such as alginic acid or corn starch; lubricants such as magnesium stearate; slip agents such as colloidal silicon dioxide; sweetening agents such as sucrose or saccharin; or flavouring agents such as mint or methyl salicylate.

Another preferred embodiment relates to the pharmaceutical composition, which further comprises a pharmaceutically acceptable excipient.

The term "excipient" refers to any substance that helps in the absorption, distribution or action of any of the active principles of the present invention, stabilises said active substance or helps in the elaboration of the medicament in the sense of giving it consistency or contributing flavours that make it more pleasant. Thus, the excipients may have the function to maintain the ingredients bound, such as, for example, starches, sugars or celluloses; a sweetening function; a colouring function; a function to protect the medicament, such as, for example, insulating it from the air and/or humidity; a filler function in a tablet, capsule or any other form of presentation, such as, for example, calcium phosphate dibasic; a disintegrating function to facilitate dissolution of the components and the absorption thereof in the intestine, without excluding other types of excipients not mentioned in this paragraph.

The term "pharmaceutically acceptable" excipient refers to the fact that the excipient is permitted and evaluated, such that it does not cause any harm to the organisms whereto it is administered.

Moreover, the excipient must be pharmaceutically adequate, i.e. it must be an excipient that allows for the activity of the active principle or active principles, i.e. it must be compatible with the active principle; in this case, the active principle is any of the compounds as described herein.

Another additional preferred embodiment relates to the pharmaceutical composition, which further comprises a pharmaceutically acceptable vehicle.

A "pharmaceutically acceptable vehicle" refers to those substances, or combination of substances, known in the pharmaceutical sector, used in the elaboration of pharmaceutical administration forms, and includes, without being limited thereto, solids, liquids, solvents or surfactants.

Like the excipient, the vehicle is a substance that is used in the medicament in order to dilute any of the compounds of the present invention to a given volume or weight. The pharmaceutically acceptable vehicle is an inert substance or a substance with an action analogous to that of any of the cells of the present disclosure. The function of the vehicle is to facilitate the incorporation of other compounds, allowing for a better dosage and administration, or give consistency and shape to the pharmaceutical composition. When the form of presentation is liquid, the pharmaceutically acceptable vehicle is the diluent.

In another preferred embodiment of the disclosure, the pharmaceutical composition further comprises another active principle. This active substance must allow for the activity of any of the compounds described herein, i.e. it must be compatible.

The pharmaceutical compositions of the present disclosure may be formulated to be administered in a variety of forms known in the state of the art.

In each case, the form of presentation of the pharmaceutical composition will be adapted to the type of administration used; for this reason, the composition of the present disclosure may be presented in the form of solutions or any other clinically permitted administration form, and in a therapeutically effective quantity.

The pharmaceutical composition of the present disclosure may be associated with, for example, without being limited thereto, liposomes or micelles. A liposome is a spherical vesicle with a phospholipid membrane. Liposomes contain a nucleus in aqueous solution. A micelle is a spherical lipid that contains non-aqueous material. Both liposomes and micelles may be used to transport various substances between the exterior and the interior of a cell. The pharmaceutical composition of the present disclosure may be associated with, for example, without being limited thereto, microcapsules of soluble polysaccharides that form spherical vesicles with an envelope made of hydrated polysaccharide, which may be used to transport various substances that cannot be mixed with one another from the exterior to the interior of the body and its organs.

The pharmaceutical compositions described herein may be used in a treatment method separately or jointly with other pharmaceutical compounds.

The term "medicament", as used in this specification, refers to any substance used for the prevention, diagnosis, relief, treatment or cure of diseases in human beings and animals. Within the context of the present invention, the disease is Fragile X syndrome; preferably, they are the symptoms of Fragile X syndrome; more preferably, they are cardiac, neurological, motor, morphological symptoms, or any combination thereof, and, even more preferably, they are cognitive and behavioural disorders.

Such compositions or combined preparations and/or the formulations thereof may be administered to an animal, including a mammal and, therefore, a human being, in a variety of forms, including, without being limited thereto, intraperitoneal, intravenous, intramuscular, subcutaneous, intrathecal, intraventricular, oral, enteral, parenteral, intranasal or dermal.

The dosage to obtain a therapeutically effective quantity depends on a variety of factors, such as, for example, the age, weight, sex, tolerance, etc. of the mammal. In the sense used in this description, the expression "therapeutically effective quantity" refers to the quantity of lipophilic antioxidant compounds and hydrophilic antioxidant compounds that produce the desired effect and, in general, will be determined, amongst other causes, by the characteristics of said active principles, prodrugs, derivatives or analogues, and by the therapeutic effect to be achieved. Preferably, the dose is 10 mg/kg/day of each compound, with a maximum of 600 mg/day for the lipophilic antioxidant compounds and a maximum of 800 mg/day for the hydrophilic antioxidant compounds. The "adjuvants" and "pharmaceutically acceptable vehicles" that may be used in said compositions are the vehicles known to persons skilled in the art.

In this specification, "Ascorbic Acid" or "Vitamin C" is understood to mean the L-enantiomer of ascorbic acid, with the formula (I):

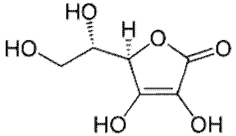

It is an essential nutrient for humans and a limited number of other species. The presence of this vitamin is required for a certain number of metabolic reactions in all animals and plants, and is internally created by almost all organisms, humans being a notable exception. Its deficiency causes scurvy in humans, hence the name ascorbic given to the acid. In living bodies, ascorbate is an antioxidant, since it protects the body against oxidation, and it is a cofactor in several vital enzymatic reactions. In humans, vitamin C is a potent antioxidant, which acts to reduce oxidative stress; and it is a substrate for ascorbate-peroxidase. This Vitamin acts as a necessary cofactor in enzymatic reactions such as, for example: enzymes that participate in the hydroxylation of collagen. Thus, vitamin C becomes an essential nutrient for the development and maintenance of scar tissue, blood vessels, and cartilages. Moreover, enzymes necessary for the synthesis of carnitine, which acts in the transport of fatty acids towards the mitochondrion for the generation of ATP. Enzymes that participate in the biosynthesis of norepinephrine from dopamine through the dopamine-beta-hydroxylase enzyme. Another enzyme adds amide groups to peptide hormones, thereby greatly increasing the stability thereof. It also acts as a cofactor in the metabolism of tyrosine. The biological tissues that acumulate more than 100 times the blood level of vitamin C are the adrenal glands, pituitary gland, thymus, corpus luteum, and retina. Those with 10 to 50 times the concentration present in the plasma include the brain, spleen, lung, testicles, lymph nodes, small intestine mucous membrane, leukocytes, pancreas, kidney and salivary glands.

Vitamin C helps in the development of the teeth and gums, bones, cartilages, in the absorption of iron, in the growth and repair of normal connective tissue (softer skin, due to the binding of the cells that need this vitamin to bind), in the production of collagen (acting as a cofactor in the hydroxylation of the amino acids lysine and proline), metabolisation of fats, healing of wounds. It has been recently described that part of the antioxidant activity of Vitamin C might be due to the inhibition of the activity of NADPH oxidase in animal models.

Human beings seem to be extremely efficient in the re-use of vitamin C, for which reason their requirements are 50 times lower than in the rest of simians. Since it is a water-soluble vitamin, its elimination by the kidney through diuresis is extremely efficient; for this reason, the excess may be eliminated in less than four hours. The United States Academy of Science recommends an intake of 60-95 milligrams of vitamin C per day in order to stay healthy. According to this organisation, 2000 milligrams per day should not be exceeded. The half-life of vitamin C is 16 days, and its half-life decreases in persons with high levels of this compound.

Vitamin C has demonstrated its utility as a treatment for the symptoms generated by Autism. A 1993 study, in 18 children with Autism, found a significant decrease of some symptoms with the treatment with vitamin C (Dolske et al. 1993).

In this specification, alpha-Tocopherol (Vitamin E) is understood to mean a compound with the formula (II):

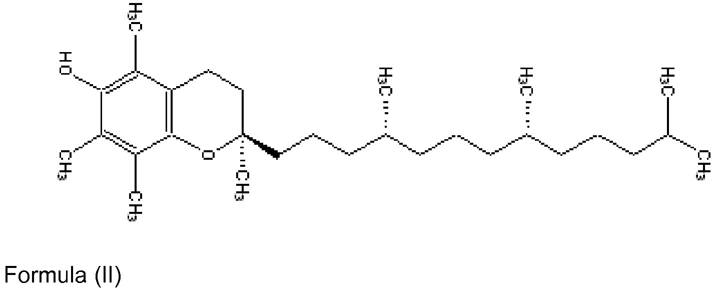

Vitamin E, or alpha-tocopherol, is a non-enzymatic liposoluble antioxidant, which may act as an indicator of the oxidative state of cells, limiting the propagation of the lipid peroxidation chain reaction. It is the main lipophilic antioxidant in the brain and significantly prevents the oxidation of lipids, which are present in high levels in the brain and must be protected by antioxidants. It has also been observed that it offers significant protection against the cytotoxicity induced by L-buthionine-sulfoximine (BSO), which causes the decrease in intracellular glutathione. Vitamin E deficiency is generally characterised by neurological disorders due to a bad transmission of nervous impulses. The use of antioxidants such as Vitamin E in the treatment of diseases related to oxidative stress, such as Alzheimer's disease, or Parkinson's disease, has shown that it is capable of delaying mental deterioration in patients with Alzheimer's disease. Moreover, vitamin E reduced lipid peroxidation and amyloid deposition in a transgenic mouse model for Alzheimer's disease.

Vitamin E develops antioxidant activity in cephalorachidian fluid (CRF) lipoproteins in the presence of physiologically relevant quantities of oxidants. Interestingly, the levels of vitamin E are reduced in the CRF extracted from patients who suffer from Alzheimer's disease and these levels increase in patients who have been treated with vitamin E supplements.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following examples and drawings are provided for illustrative purposes, and are not intended to limit the scope of the present invention.

In this specification, vitamin A (retinol or vitamin A, retinal, and four carotenoids, including beta-carotene) is understood to mean a liposoluble vitamin; it helps in the formation and maintenance of healthy teeth, soft and bone tissues, mucous membranes and the skin. Vitamin A (formula III) is an essential nutrient for human beings. It is also known as retinol, since it generates necessary pigments for the functioning of the retina, and also as an acid (retinoic acid). It plays a significant role in the development of good vision, especially in faint light (scotopic vision). It may also be required for reproduction and breast-feeding. β-carotene, which has antioxidant properties, is a precursor of vitamin A. Vitamin A also acts in a very different role as an irreversible oxidised form of retinol known as retinoic acid, which is a hormone, and as an important growth factor for epithelial cells and other structures.

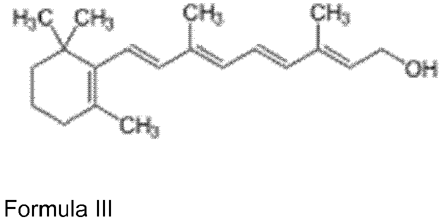

### DESCRIPTION OF THE FIGURES

**Fig. 1****. Open-field distance travelled by the mice in a 15-minute period of time for the different treatments.** FMR1-KO n = 10 (black bars) and WT n = 11 (white bars).* p < 0.05 FMR1-KO vs. WT. ** p < 0.01 FMR1-KO vs. WT. # p < 0.05 FMR1-KO VitE-Cys, Melatonin, and VitE vs. FMR1-KO without treatment, saline control and Tianeptine.
**Fig. 2****. Percentage permanence time in the open arm of the raised cross-shaped maze in a 5-minute period of time for the different treatments.** FMR1-KO (n = 10): black bars, WT (n = 11): white bars. * p < 0.05 FMR1-KO vs. WT. # p < 0.05 FMR1-KO VitE-Cys, Melatonin, and VitE vs. FMR1-KO without treatment, saline control and Tianeptine.
**Fig. 3****. Concentration of corticosterone in mouse serum (ng/ml).** FMR1-KO (n = 10): black bars and WT (n = 11): white bars. * p < 0.05 FMR1-KO vs. WT. ** p < 0.01 FMR1-KO vs. WT. # p < 0.05 FMR1-KO Treated vs. FMR1-KO Saline Control.
**Fig. 4****. Concentration of TBARS in mouse cerebral membranes (nmol/mg protein).** FMR1-KO (n = 10): black bars and WT (n = 11): white bars. * p < 0.05 FMR1-KO vs. WT. # p < 0.05 FMR1-KO vs. FMR1-KO. † p < 0.05 WT vs. WT.
**Fig. 5****. Concentration of GSH in mouse brain extract (µmol/g protein).** FMR1-KO (n = 8): black bars and WT (n = 9): white bars. * p < 0.05 FMR1-KO vs. WT. # p < 0.05 FMR1-KO vs. FMR1-KO. † p < 0.05 WT vs. WT.
**Fig. 6****. Percentage of GSSG with respect to GSH in mouse brain extract.** FMR1-KO (n = 8): black bars and WT (n = 9): white bars. * p < 0.05 FMR1-KO vs. WT. # p < 0.05 FMR1-KO vs. FMR1-KO. † p < 0.05 WT vs. WT.

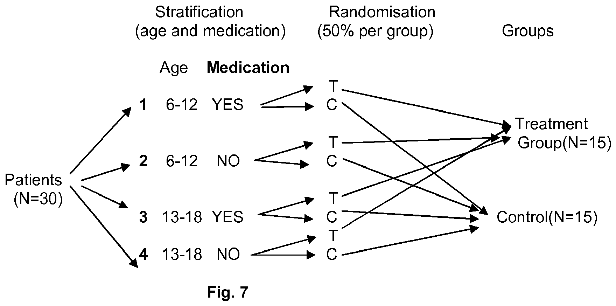

**Fig. 7****. Randomisation criterion for the FXS-antioxidants trial.** Blocked randomisation and stratification by factors of confusion (age and concomitant medication).

### EXAMPLES

Below we will illustrate the invention by means of trials performed by the inventors.

### Pre-clinical trials

*Treatment trials in the model mouse for Fragile X Syndrome in order to re-balance the oxidation-reduction state using different compounds that are neuroprotectors and regulate suprarenal secretion, tocopherol and melatonin.*
- Oral and IP chronic treatments in the FMR1 mouse using the following compounds: Melatonin (100 mg/kg/day), Vitamin E (100 mg/kg/day), Cysteine (200 mg/kg/day), Clonidine (2 mg/kg/day), Tianeptine (10 mg/kg/day), MPEP (2 mg/kg/day), Quercetin (10 mg/kg/day).

In order to study the effectiveness of the treatments performed on the groups of experimental mice, a number of verifications were performed at the behavioural level and the biochemical level. Below we indicate the results for each section.

### 1. Behavioural studies

### 1.1. Open field

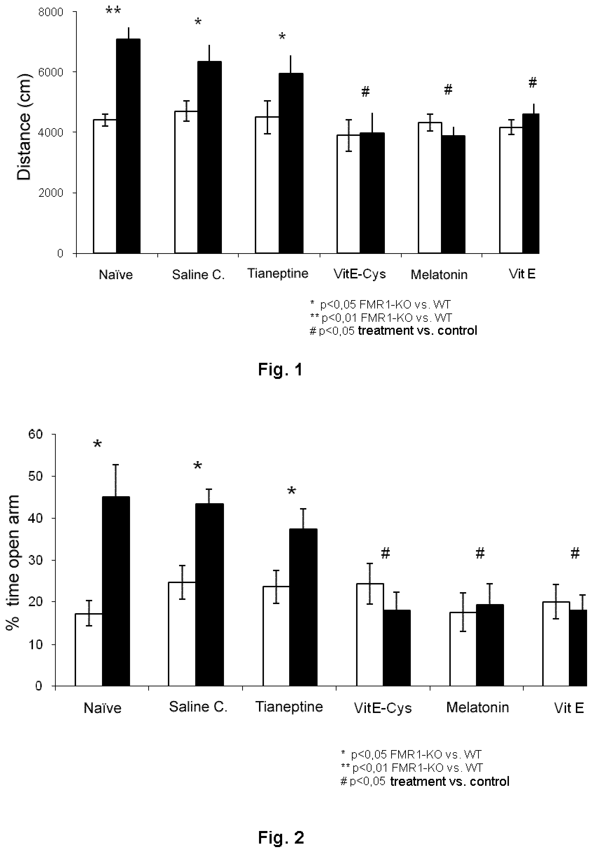

In the open-field, locomotive activity, a greater total travelled distance was observed in the mice with the FMR1-KO genotype as compared to the WT control group, in naive and saline control mice, accentuated under naive conditions (p < 0.01), as compared to the saline control group (p < 0.05) and the group under treatment with tianeptine (p < 0.05). The total distance travelled decreased in FMR1-KO mice in the rest of the treatments, VitE-Cys, Melatonin and VitE, equalling the distances travelled by the WT control group. In the FMR1-KO mice with the treatments with VitE-Cys, Melatonin, and VitE, the distance travelled was lower (p < 0.05) than in the animals without treatment, the saline control and the Tianeptine animals. In all the groups of WT mice, locomotion was maintained without significant variations, about 4000 cm of distance travelled (Figure 1); this test was used to check the mice's hyperactivity.

### 1. 2. Raised cross-shaped maze

In the FMR1-KO mice in the naive group without treatment, and also in the saline control and Tianeptine groups, the percentage permanence time in the open arm of the maze was greater (p < 0.05) than in the group of WT control mice. As regards the treatments with VitE-Cys, Melatonin, and VitE, in no case were significant differences observed in the permanence time in the open arm. There was also no variation in the permanence times between the WT control groups in any of the groups. In the groups of FMR1-KO mice treated with VitE-Cys, Melatonin, and VitE, the permanence time in the open arm was lower (p < 0.05) than in the animals without treatment, the saline control and the Tianeptine animals (Figure 2). This test is used to determine the anxiety of the animal model, under baseline and treated conditions.

### 2. Biochemical parameters

### 2. a. Measurement of corticosterone in serum

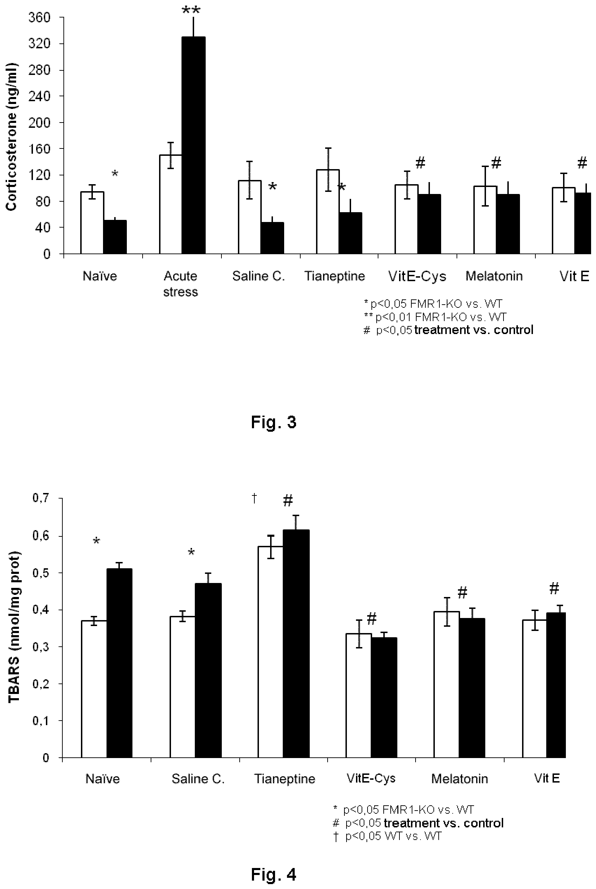

In the animals that were subjected to an acute stress (simple saline solution IP injection), a marked increase was observed in serum corticosterone levels in the FMR1-KO mice (330 ± 35 ng/ml) as compared to the WT mice (150 ± 20 ng/ml). The concentration of corticosterone in the serum of FMR1-KO mice in the naive, saline control and Tianeptine groups was lower than in the serum of the groups of WT control mice. However, the treatment with Tianeptine was not capable of equalling the levels of this hormone in the animals, and concentrations of corticosterone similar to that specified in the saline control group were observed in the mice. On the contrary, in the groups of mice treated with VitE-Cys, Melatonin and VitE, it was observed that the serum of FMR1-KO mice maintained the same levels of corticosterone as compared to the WT controls under treatment (Figure 3).

### 2. b. Measurement of the stress hormone content in the suprarenal gland

In the study animals, the suprarenal hormones were characterised in the Fmr1-KO mouse. The mouse model presents greater adrenalin content in the suprarenal glands than the WT controls (Table 1).

**Table 1. Catecholamine content in the adrenal gland (ng/mg of tissue)**

| | Control-WT (n = 10) | Fmr-1KO (n = 10) |
|---|---|---|
| **Adrenalin** | **1214.84 ± 234.7** | **1718.46 ± 116.63*** |
| Noradrenalin | 489.02 ± 115.6 | 639.54 ± 55.64 |
| Dopamine | 24.62 ± 5.65 | 29.44 ± 3.01 |

| | | |
|---|---|---|
| Data = Mean ± SEM *p < 0.05 Fmr1-KO versus Control-WT | | |

### 2. c. Determination of the level of lipid peroxidation

The levels of TBARS were observed to be high in the FMR1-KO mice as compared to the WT groups in the saline and naive controls. The treatment with VitE-Cys, Melatonin and Vitamin E normalises the levels of TBARS between the different treatments as compared to the control group. However, in the animals treated with tianeptine, an increase in the values of TBARS was observed in the two genotypes with respect to the saline control group, whereas amongst FMR1-KO mice these values were higher than for the treatment with Vitamin E, VitE-Cys and Melatonin (Figure 4).

### II. GSH-GSSG redox state

### Reduced glutathione (GSH)

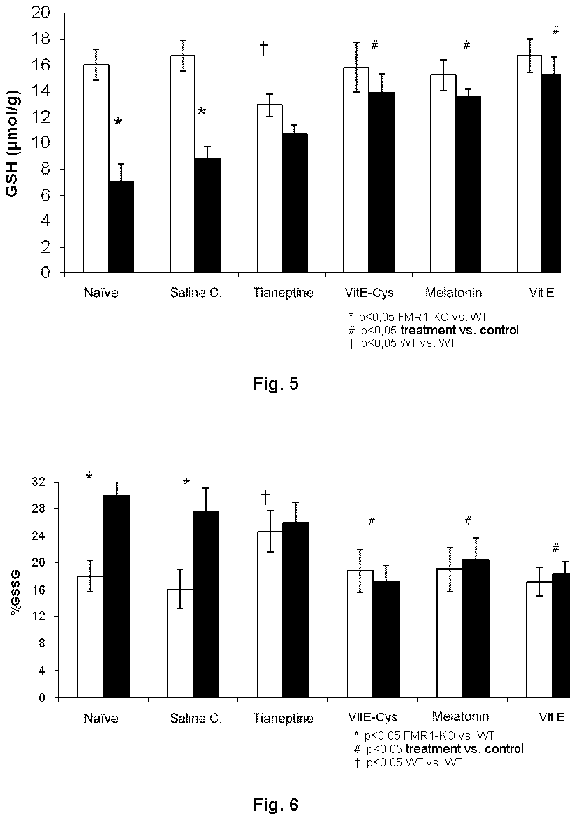

In the group of saline control FMR1-KO mice, a decrease in the quantity of GSH was observed as compared to WT mice. The FMR1-KO mice treated with VitE-Cys, Melatonin and VitaminE show higher values of GSH than those in the saline and Tianeptine controls, normalised with respect to the treated control groups. In the treatment with Tianeptine, no significant differences were found in the groups of FMR1-KO mice with respect to the saline control. On the contrary, the WT mice showed a decrease in the value of GSH with respect to the rest of groups of treated animals (Figure 5).

### Oxidised glutathione (GSSG)

In the saline and naive control groups of FMR1-KO mice, the percentages of GSSG with respect to GSH were higher than in the WT control groups, whereas no significant differences were found in the rest of the groups. The treatments with VitE-Cys, Melatonin and VitaminE of groups of FMR1-KO mice normalised the values of GSSG as compared to the saline and naive controls. By comparison with the WT mice, a significant difference (higher percentage) with respect to the rest of the groups was only found amongst those treated with Tianeptine (Figure 6).

### II.e. Evaluation of blood parameters in patients.

Characterisation, in a group of 17 patients and 15 healthy controls (siblings of the patients), of different blood parameters, such as levels of vitamins, suprarenal hormones and different hormonal axes, related to manifestations of the patients affected.

**Table 3. Analysis of the data of the hormonal and biochemical studies in XF patients and their healthy siblings as controls**

| | Age (years) | Adrenalin (ng/l) | Noradrenalin (ng/l) | Cortisol (mcg/dl) | Serotonin (ng/ml) | Corpus Hb (pg) | cAMP (nmol/l) | Vitamin E (mg/l) | Vitamin C (mg/dl) |
|---|---|---|---|---|---|---|---|---|---|
| Controls (n = 17) | 13.50 | 45.18 | 336.67 | 11.80 | 78.83 | 27.20 | 48.82 | 9.45 | 0.76 |
| Affected FXS (n = 15) | 11.20 | 207.3** | 647.2* | 11.70 | 83.30 | 26.16 | 45.72 | 8.95 | 0.32* |
| *SEM controls* | *4.99* | *14.37* | *68.97* | *2.13* | *28.67* | *1.21* | *8.71* | *0.96* | *0.13* |
| *SEM FXS* | *3.08* | *37.89* | *89.22* | *1.82* | *29*.46 | *0.53* | *3.36* | *0.90* | *0.16* |
| Data = Mean *p > 0.05 **p < 0.005 FXS versus Control | | | | | | | | | |

The levels of the suprarenal hormones adrenalin and noradrenalin are significantly high in blood samples from patients affected with FXS as compared to samples from healthy controls. There is also a reduction in the plasma levels of vitamin C.

### Clinical trials in patients

One-way cross-over placebo-controlled, double-blind, randomised clinical trial (phase II), with 2 12-week-long treatment periods.

*Subjects:* Males between 6-18 years of age with Fragile X Syndrome. Male patients affected by Fragile X Syndrome, diagnosed by means of DNA molecular genetic study, and grouped into two categories: Children (6-12 years of age) and adolescents (13-18 years of age), seen at the Maternal and Child- Carlos Haya Hospital in Málaga. Participants in the study, 30 patients.

*Intervention:* 30 participants randomly assigned to receive the antioxidant combination of vitamin C (ascorbic acid 10 mg/kg/day) and vitamin E (d-alpha-tocopherol 10 mg/kg/day) twice a day or placebo during the first 12-week phase. All the participants received the antioxidants vitamin C (ascorbic acid 10 mg/kg/day) and vitamin E (d-alpha-tocopherol 10 mg/kg/day) twice a day for 12 weeks during the second phase.

### Inclusion criteria:

- Molecular genetic diagnosis of the syndrome (no. of CGG repeats in the FMR1 gene greater than 200).
- Presenting symptoms characteristic of Fragile X Syndrome.
- Being male
- Being between 6 and 18 years of age
- Having the informed consent document signed by the parents and/or tutors prior to initiating their participation in the trial.
- Both the parents and the affected patients must commit to participating in the trial for 20 weeks.

### Exclusion criteria:

- Those individuals with other neurological disorders not related to the syndrome were excluded from the study.
- Having had serious medical problems in the last 12 months.
- Taking 100 mg per day of vitamins E or C in the last 4 months.
- Having physical, psychic or sensory problems that prevent evaluation of the effectiveness.
- Hypersensitivity to any of the components in the preparation.

### DESCRIPTION OF THE TREATMENT.

Doses:
- Vitamin E (Tocopherol): 10 mg/kg/day (Maximum of 600 mg/day)
- Vitamin C (Ascorbic Acid): 10 mg//kg/day (Maximum of 800 mg/day)

Treatment group: two daily doses of the aformentioned combination.
- Administered at breakfast and dinner

Placebo group: two daily doses of the placebo.
- Administered at breakfast and dinner

Duration of the treatment: 28 weeks: One week for the initial or baseline evaluation, a first 12-week phase wherein the participants received treatment or placebo, randomly assigned by the Pharmacy Service of the Carlos Haya Hospital, one week for the 2nd evaluation, a second 12-week phase wherein all the participants received treatment and, finally, one week for the 3rd evaluation.

### DEVELOPMENT OF THE TRIAL AND EVALUATION OF THE RESPONSE

EVALUATION OF THE SAFETY: Prior to beginning and after completing the treatment, the following studies were performed:
A) Haematological: Haemoglobin (corpuscular haemoglobin), haematocrit, RBC, WBC, platelets.
B) Biochemical: Creatinine, urea, glucose, uric acid, sodium, potassium, calcium, phosphate, chlorine, total protein, albumin, triglycerides, total cholesterol, HDL, total bilirubin, AST (SGOT), ALT (SGPT), gamma GT, alkaline phosphatase, cAMP, glutamic acid, pyruvic acid.
C) Determination of the suprarrenal Axis (Adrenalin, Noradrenalin, Dopamine, Cortisol, ACTH): baseline and under stress, in blood, urine (with its metabolites), saliva (cortisol).
D) Oxidative status in blood: MDA, Glutathione (total, reduced, oxidised) Antioxidant system enzymes: glutathione peroxidase (GPx), glutathione reductase, glutathione transferase. Superoxide dismutase (SOD), Catalase. Plasma F2-isoprostanes. Total antioxidant status (TAS) and DNA damage by the Comet assay. Levels of oxidation in proteins (Carbonyls),
E) Others determinations: Hormones (LH/FSH/GH/TSH/T4) Neurotransmitters: (Serotonin/GABA). cAMP. Vitamins (Vitamin A/Vitamin C/Vitamin E). Ceruloplasmin/Albumin/Transferrin. Minerals: Selenium/Zinc/Copper/Manganese. Determination of amino acids in serum.
F) Urine test: density, PH, proteins, glucose, ketone bodies, bilirubin, blood, nitrites, urobilinogen, leukocytes, urinary sediment, sodium, chlorine, potassium.

### MEDICAL PARAMETERS TO BE EVALUATED IN THE SYNDROME.

A) CARDIAC SYMPTOMS: Mitral valve prolapse.
B) NEUROLOGICAL SYMPTOMS: Epilepsy.
C) MOTOR SYMPTOMS: Hypotonia. Gross and fine motor delay. Stereotipies. Joint hypermobility.
D) MORPHOLOGICAL: Macroorchidism. Scoliosis. Hyperpigmentation of the skin. Otitis. Strabismus. Macrencephaly.

### MAIN EVALUATION OF COGNITIVE AND BEHAVIOURAL DISORDERS.

The following primary instruments were used to evaluate behaviour and hyperactivity: "Developmental behaviour checklist for parents (DBC-P) and for teachers (DBC-T)" and "Conners questionnaire revised for parents and teachers" (CRS-R), and to evaluate learning: "Wechsler intelligence scale revised for children"; at 0, 3 and 6 months during the trial and 3 months after ending the treatment.

Evaluations to be performed by the parents:
- Journal of events.
- Questionnaire about nutrition.
- BASC-II adaptive behaviour assessment system. Form for parents in Spanish.
- Treatment checklist (ATEC). Autism Treatment Evaluation Checklist.
- Conners questionnaire for parents, which will be filled prior to beginning the treatment and every 4 weeks after initiating the treatment.
- Developmental behaviour checklist (DBC-P24).

Evaluations to be performed by teachers at school and therapists:
- Journal of events.
- BASC-II adaptive behaviour assessment system. Form for caregivers in Spanish.
- Treatment checklist (ATEC). Autism Treatment Evaluation Checklist.
- Conners questionnaire for teachers, which will be filled prior to beginning the treatment and every 4 weeks after initiating the treatment.
- Developmental behaviour checklist (DBC-P24).

Since this a low-frequency disease, with 1 male in 2500 affected, a computer programme was used to estimate that a minimum number of 30 patients should be included in the trial in order to have a sufficiently large population to obtain significant results. In the data analysis, the type of treatment (antioxidant/placebo) was considered to be the independent variable, and the dependent variables were the percent change in the different behavioural and learning tests, the percent change in the biochemical tests, the percent change in the blood levels of antioxidants, as well as the adverse reactions in each group. Fischer's exact test was used to compare the percentages and Mann-Whitney's U test was used for the numerical variables. A difference was considered to be statistically significant for values of p<0.05. The information was processed using the SPSS 14.0 for Windows statistical package.

All the medicaments and complementary therapies remained constant throughout the entire study. Before the beginning of the study, the participants had a first blood test in order to determine the baseline situation, and the test was repeated upon ending the study. Questions will be asked regarding nutrition and special diets and what types of medicines they have used and the effectiveness thereof. The study had two phases.

TREATMENT ADMINISTERED: The study participants received vitamins E+C or placebo in the first phase of the study. In the second phase they all received vitamins E+C in the study. The selection of the patients in each group, who received the placebo or vitamins E+C, was randomly decided. The study participants took vitamins E+C or placebo twice a day. The study participants and the evaluating physicians did not know whether a participant was taking vitamins E+C or the placebo.

The study participants came to the centre responsible for the trial 3 times, for 4-6 hours the first time, and for 3-4 hours during the 2 subsequent visits. A comprehensive medical examination was performed, and adaptive skills questionnaires and behavioural and learning tests were given. A blood test was performed in the three visits in order to study the effectiveness parameters. Daily notes were taken by the family and the caregivers and teachers throughout the entire study. The needle punctures to extract venous blood usually do not cause serious problems; however, a slight discomfort may appear in the puncture area, such as pain or bleeding.

*RESULT MEASUREMENTS:* Level of oxidative stress (indicated by the level of glutathione and the oxidation levels of lipids and proteins) and suprarenal hormonal response. Studies were also performed in order to analyse the improvement in the participants' behaviour, through the following instruments: "Developmental behaviour checklist (DBC-P and DBC-T)" and "Conners questionnaire for parents and teachers"; as well as learning measurements using the instrument "Wechsler intelligence scale revised for children"; at 0, 3 and 6 months during the trial.

**Table 1. Demographic sample in the antioxidant phase-II trial for patients with Fragile X Syndrome between 6 and 18 years of age.**

| **Patient characteristics** | **Active (n=15)** | **Placebo (n =15)** | **Total (n= 30)** |
|---|---|---|---|
| **Age, years, M (SD)** | 12.1 (3.4) | 11.7 (4.8) | 11.6 (4.2) |
| **Age groups, *n* (%)** | | | |
| 6-12 years | 7 (56) | 8 (44) | 15 (50) |
| 13-18 years | 8 (37) | 7 (63) | 15 (50) |
| **Gender, n (%)** | | | |
| Male | 15 (50) | 15 (50) | 30 (100) |
| **Weight M (SD)** | 53.2 (6.6) | 50.9 (6.6) | 52.1 (4.6) |
| **Psychopharmacological treatment (%)** | 11 (36.66) | 7 (23.3) | 18 (60) |
| **Conners questionnaire revised for parents M (SD)** | | | |
| DSM-IV Hyperactive/impulsive | 67.8 (12.1) | 64.2 (12.0) | 66.0 (12.0) |
| DSM-IV Inattentive | 60.3 (8.8) | 62.2 (9.3) | 61.3 (9.0) |
| **Conners questionnaire revised for teachers, M (SD)** | | | |
| DSM-IV Hyperactive/impulsive | 62.8 (10.3) | 64.7 (14.4) | 63.7 (12.3) |
| DSM-IV Inattentive | 64.6 (6.7) | 67.6 (8.1) | 66.1 (7.5) |
| **Associated conditions, *n* (%)** | | | |
| Epilepsy | 1 (3.3) | 0 (0) | 1 (3.3) |
| Asthma | 2 (6.6) | 0 (0) | 2 (6.6) |
| Autistic symptoms | 2 (6.6) | 2 (6.6) | 4 (13.2) |
| Obsessive-compulsive disorders | 0 (0) | 1 (3.3) | 1 (3.3) |

| | | | |
|---|---|---|---|
| Note: DSM-IV = Diagnostic Manual of Mental Disorders, 4th Edition; M=Mean; SD=Standard deviation; n=number of patients; p=statistical significance; %=percentage. | | | |

**Table 2. Patient characteristics and response rates in subgroups following 12 weeks of treatments with an antioxidant combination (vitamins C and E) or placebo.**

| | (Number of respondent patients after 12 weeks of treatment/patients with symptoms) | | | |
|---|---|---|---|---|
| **Patient Characteristics** | **n (initial)** | **Antioxidant** | **Placebo** | **P** |
| **Patients** | 30 | (11/15) | (4/15) | *04* |
| **Age groups** | | | | |
| 6-12 years | 15 | (7/8) | (1/8) | 04 |
| 13-18 years | 15 | (4/7) | (3/7) | ns |
| **Conners questionnaire revised for parents** | | | | |
| DSM-IV Hyperactive/impulsive | 22 | (7/10) | (2/12) | *04* |
| DSM-IV Inattentive | 21 | (5/9) | (4/12) | *ns* |
| **Conners questionnaire revised for teachers** | | | | |
| DSM-IV Hyperactive/impulsive | 23 | (5/12) | (2/11) | *ns* |
| DSM-IV Inattentive | 26 | (8/13) | (2/13) | *ns* |

| | | | | |
|---|---|---|---|---|
| Note: DSM-IV = Diagnostic Manual of Mental Disorders, 4th Edition; M = Mean; SD=Standard deviation; n=number of patients; p=statistical significance; %=percentage. | | | | |

## Claims

1. A pharmaceutical composition or a combined pharmaceutical preparation wherein tocopherol (vitamin E) and ascorbic acid (vitamin C) are the sole active principles, for use in the treatment of fragile X syndrome in a human.

2. The pharmaceutical composition or the combined pharmaceutical preparation for use according to claim 1, in the treatment of the behavioural and cognitive disorders associated to fragile X syndrome in said human.

3. The pharmaceutical composition for use according to any of claims 1 or 2, wherein the pharmaceutical composition further comprises pharmaceutically acceptable vehicles and/or excipients.

4. The combined pharmaceutical preparation for use according to any of claims 1 or 2, wherein the active principles of the combined pharmaceutical preparation are administered sequentially.

5. The pharmaceutical composition or the combined pharmaceutical preparation for use according to any of claims 1 to 4, wherein the human is under 20 years of age.

6. The pharmaceutical composition or the combined pharmaceutical preparation for use according to claim 5, wherein the human is between 6 and 12 years of age.

7. The pharmaceutical composition or the combined pharmaceutical preparation for use according to any of the precedent claims, wherein said composition or preparation is administered at a dose of about 10 mg/kg/day of each of tocopherol and ascorbic acid, with a maximum of 600 mg/day for tocopherol and a maximum of 800 mg/day for ascorbic acid.

8. The pharmaceutical composition or the combined pharmaceutical preparation for use according to any of the precedent claims, wherein each of tocopherol and ascorbic acid are administered once or twice per day.

## Patentansprüche

1. Pharmazeutische Zusammensetzung oder kombinierte pharmazeutische Zubereitung, in welcher Tocopherol (Vitamin E) und Ascorbinsäure (Vitamin C) die einzigen Wirkstoffe sind, für deren Verwendung bei der Behandlung von fragilem X-Syndrom in einem Menschen.

2. Pharmazeutische Zusammensetzung oder kombinierte pharmazeutische Zubereitung für deren Verwendung nach Anspruch 1, bei der Behandlung von kognitiven und Verhaltensstörungen, welche mit dem fragilem X-Syndrom assoziiert sind, im genannten Menschen.

3. Pharmazeutische Zusammensetzung für deren Verwendung nach einem der Ansprüche 1 oder 2, wobei die pharmazeutische Zusammensetzung zusätzlich pharmazeutisch akzeptable Träger und/oder Hilfsstoffe umfasst.

4. Kombinierte pharmazeutische Zubereitung für deren Verwendung nach einem der Ansprüche 1 oder 2, wobei die Wirkstoffe der kombinierten pharmazeutischen Zubereitung sequenziell verabreicht werden.

5. Pharmazeutische Zusammensetzung oder kombinierte pharmazeutische Zubereitung für deren Verwendung nach einem der Ansprüche 1 bis 4, wobei der Mensch unter 20 Jahre alt ist.

6. Pharmazeutische Zusammensetzung oder kombinierte pharmazeutische Zubereitung für deren Verwendung nach Anspruch 5, wobei der Mensch zwischen 6 und 12 Jahre alt ist.

7. Pharmazeutische Zusammensetzung oder kombinierte pharmazeutische Zubereitung für deren Verwendung nach einem der vorhergehenden Ansprüche, wobei die genannte Zusammensetzung oder Zubereitung bei einer Dosis von etwa 10 mg/kg/Tag von jedem aus Tocopherol und Ascorbinsäure, mit einem Maximalwert von 600 mg/Tag für Tocopherol und einem Maximalwert von 800 mg/Tag für Ascorbinsäure, verabreicht wird.

8. Pharmazeutische Zusammensetzung oder kombinierte pharmazeutische Zubereitung für deren Verwendung nach einem der vorhergehenden Ansprüche, wobei jedes aus Tocopherol und Ascorbinsäure einmal oder zweimal pro Tag verabreicht wird.

## Revendications

1. Composition pharmaceutique ou préparation pharmaceutique combinée dans laquelle le tocophérol (vitamine E) et l'acide ascorbique (vitamine C) sont les seuls principes actifs, pour une utilisation dans le traitement du syndrome de l'X fragile chez un être humain.

2. Composition pharmaceutique ou préparation pharmaceutique combinée pour une utilisation selon la revendication 1, dans le traitement des troubles cognitifs et du comportement associés au syndrome de l'X fragile dans ledit être humain.

3. Composition pharmaceutique pour une utilisation selon l'une quelconque des revendications 1 ou 2, dans laquelle la composition pharmaceutique comprend en outre des véhicules et/ou des excipients pharmaceutiquement acceptables.

4. Préparation pharmaceutique combinée pour une utilisation selon l'une quelconque des revendications 1 ou 2, dans laquelle les principes actifs de la préparation pharmaceutique combinée sont administrés de manière séquentielle.

5. Composition pharmaceutique ou préparation pharmaceutique combinée pour une utilisation selon l'une quelconque des revendications 1 à 4, dans laquelle l'être humain a moins de 20 ans.

6. Composition pharmaceutique ou préparation pharmaceutique combinée pour une utilisation selon la revendication 5, dans laquelle l'être humain a entre 6 et 12 ans.

7. Composition pharmaceutique ou préparation pharmaceutique combinée pour une utilisation selon l'une quelconque des revendications précédentes, dans laquelle ladite composition ou préparation est administrée à une dose d'environ 10 mg/kg/jour aussi bien de tocophérol que d'acide ascorbique, avec une quantité maximale de 600 mg/jour de tocophérol et une quantité maximale de 800 mg/jour d'acide ascorbique.

8. Composition pharmaceutique ou préparation pharmaceutique combinée pour une utilisation selon l'une quelconque des revendications précédentes, dans laquelle aussi bien le tocophérol que l'acide ascorbique sont administrés une ou deux fois par jour.
